# EUROPEAN PATENT APPLICATION

(11) **EP 3 613 856 A1**
(43) Date of publication of application: **26.02.2020**
(21) Application number: 18777944.2
(22) Date of filing: 26.03.2018
(51) Int. Cl.: C12N 15/113, C12N 15/864, C12N 7/00, C12N 5/10, A61K 48/00, A61K 31/713, A61K 35/76, A61K 39/23, A61P 21/00, A61P 31/18, A61P 31/20, A61P 9/10

(54) **SHRNA EXPRESSION CASSETTE, POLYNUCLEOTIDE SEQUENCE CARRYING SAME, AND APPLICATION THEREOF**

(30) Priority: 31.03.2017 CN 201710210863
(71) Applicant: Staidson(Beijing) Biopharmaceuticals Co., Ltd., Beijing 100176 (CN); Beijing SoloBio Genetechnology Company Ltd., Beijing 100176 (CN)
(72) Inventor: WANG, Chao, Beijing 100176 (CN); ZHANG, Tingting, Beijing 100176 (CN); JIANG, Lixin, Beijing 100176 (CN)
(74) Representative: Hertin und Partner Rechts- und Patentanwälte PartG mbB
(86) International application number: PCT/CN2018/080480
(87) International publication number: WO 2018/177244

(57) **Abstract**

An shRNA expression cassette, a polynucleotide sequence carrying the same, and an use thereof. In an order of 5' to 3', the shRNA expression cassette sequentially includes a DNA sequence for expressing the shRNA and a stuffer sequence, and a sequence length of the shRNA expression cassette sequence is proximate to a length of a wild-type AAV genome.

## Description

### CROSS-REFERENCE TO RELATED APPLICATION

This application claims a priority to Chinese Patent Application No. 201710210863.5 filed on March 31, 2017, the disclosures of which are incorporated in their entirety by reference herein.

### TECHNICAL FIELD

The present disclosure relates to an shRNA expression cassette, a polynucleotide sequence carrying the same, and use thereof, and belongs to the fields of biotechnology and gene therapy.

### BACKGROUND

Small interfering RNA (siRNA), sometimes called short interfering RNA or silencing RNA, is a double-stranded RNA having 20 to 25 nucleotides in length and has many different use in biology. When the siRNA targeting the target gene is designed into a hairpin structure to be introduced into the body, the hairpin structure expresses and forms shRNA, in which shRNA is an abbreviation of short hairpin RNA, and includes two short inverted repeat sequences. It is currently known that shRNA is mainly involved in the phenomenon of RNA interference (RNAi), and regulates gene expression in a specific manner. In addition, it is also involved in some RNAi-related reaction pathways, for example antiviral mechanisms or changes in chromatin structure. After shRNA enters the cell, it is unwound by the RNA helicase in host cell into a sense RNA strand and an antisense RNA strand, in which the antisense RNA strand binds to some enzymes in the body to form a silencing complex RISC (RNA-induced silencing complex) which recognizes and combines with the mRNA containing its complementary sequence. At this time, a phenomenon, in which RISC has the function of nuclease and is capable of cleaving and degrading mRNA, thereby suppressing the expression of a corresponding gene, is called RNA interference (RNAi).

ShRNA features include gene sequence specificity, efficiency, and hereditability. ShRNA is easy to be degraded after entering the cell, and the half-life is short. It is difficult to penetrate the cell membrane and vascular endothelium, and the transfection efficiency is limited. The amount of shRNA entering the cell is not controlled, and it is easy to accumulate in the spleen. An efficient vehicle or delivery route is required to deliver exogenous shRNA into an organism. One method is to deliver directly using a chemical modification method, such as lipofection and PEG modification, and the shRNA can be directly introduced into the cell to inhibit the expression of a specific gene without cloning into a vector. The method of plasmid or viral vector-mediated expression of shRNA in vivo shows advantages over using the shRNA expression cassette directly. The dsRNA sequence corresponding to the shRNA is cloned into a plasmid vector or a viral vector containing a suitable promoter, then the cell is transfected with the plasmid or infected with the virus, and the desired shRNA is formed through transcription under the control of the promoter, and thus plays a role in sustainably generating siRNA in vivo.

Adeno-associated virus (AAV) is a member of the parvovirus family, is a non-enveloped single-stranded linear DNA virus, and has many advantages as a gene therapy vector, such as high infection efficiency, a wide range of infection, and high safety for long-term expression, etc. It is used clinically to treat tumors, retinal disease, arthritis, AIDS, heart failure, muscular dystrophy, nervous system disease, and a variety of other genetic defect diseases. However, since the shRNA involved in the present disclosure is very short, only a few tens of bp, packaging efficiency and expression amount are the problems when it is expressed using the AAV viral vector.

### SUMMARY

Some embodiments of the present disclosure provide an shRNA expression cassette, a polynucleotide sequence carrying the shRNA expression cassette, a recombinant vector plasmid carrying the polynucleotide sequence, an shRNA, a host, a virus particle, an isolated engineered cell, a pharmaceutical composition, their use in the preparation of a medicament for prevention and treatment of hepatitis B, acquired immunodeficiency syndrome, for treatment of Duchenne muscular dystrophy (DMD), and for treatment of hypercholesterolemia, and a vector preparation system for the recombinant vector plasmid.

The expression cassette of the present disclosure is expanded by using a stuffer sequence, such that the length of the expression cassette sequence is proximate to the length of the wild-type AAV genome. The expression cassette or the polynucleotide sequence carrying the same when expressed by using an AAV viral vector is capable of ensuring the yield of virus packaging. After extensive experiments, it was found that when the stuffer sequence is located at the 3' end of the shRNA sequence, the expression cassette or the polynucleotide sequence carrying the same is expressed by using the AAV viral vector, it not only guarantees the viral packaging yield, but also increases the expression level of the target gene shRNA, thereby improving the therapeutic effect of the drug.

According to an aspect of the present disclosure, the present disclosure provides an shRNA expression cassette, in which in an order of 5' to 3', the shRNA expression cassette sequentially includes a DNA sequence for expressing the shRNA and a stuffer sequence, and a sequence length of the shRNA expression cassette is proximate to a length of a wild-type AAV genome. The stuffer sequence is optionally a human non-coding sequence.

Optionally, the human non-coding sequence is inert or innocuous, and does not have function or activity. In various particular aspects, the human non-coding sequence is not a sequence encoding a protein or polypeptide, unlike any of the following substances: shRNA, AAV terminal inverted repeat (ITR) sequence, promoter, replication origin, polyadenylation sequence, and the like.

The human non-coding sequence is selected from a sequence fragment or a combination of a plurality of sequence fragments of an intron sequence of human Factor IX (GenBank: K02402.1), an intron I fragment of human Factor IX, a sequence of human cosmid C346 or the HPRT-intron sequence at positions 1704-14779 (GenBank: M26434.1); even is optionally a sequence fragment of the HPRT-intron sequence at positions 1704-14779 (GenBank: M26434.1). The length of the human non-coding sequence can be adjusted according to different AAV packaging capacity or other requirements, and is not limited to a fixed length.

Optionally, the sequence length of the expression cassette that is expressed by using a single-stranded AAV viral vector or used directly is 3.2 kb to 5.2 kb, optionally 3.8 kb to 5.1 kb, and even optionally 3.8 kb to 4.6 kb and 4.6 kb to 5.1 kb, especially optionally 4.6 kb.

Even optionally, the sequence length of the shRNA expression cassette that is expressed by using a double-stranded AAV viral vector is half of the sequence length of the shRNA expression cassette that is expressed by using the single-stranded AAV viral vector or used directly. Optionally, it is half of 3.2 kb to 5.2 kb, optionally half of 3.8 kb to 5.1 kb, or even optionally half of 3.8 kb to 4.6 kb or 4.6 kb to 5.1 kb, especially optionally half of 4.6 kb, i.e., 2.3kb.

In the shRNA expression cassette of the present disclosure, the 5' end of the DNA sequence for expressing the shRNA contains a promoter, the promoter may be a promoter from any source, and the promoter includes one or more selected from an RNA polymerase II promoter and an RNA polymerase III promoter. The RNA polymerase II promoter is a commonly used promoter, such as CAG promoter, CMV promoter, SV40 promoter, EF1 promoter, Ub promoter. As for different pathological tissues or cells, tissue or cell-specific promoters, such as LP1 promoter, CK1 promoter, DC172 promoter, DC190 promoter, ApoE/hAAT promoter, ApoA-I promoter, TBG promoter, LSP1 promoter, HD-IFN promoter, etc., may be selected according to specific pathological characteristics. In order to ensure high efficient expression of the target gene shRNA, the promoter may be a promoter or a combination of a plurality of promoters; and the promoter may be a mutant or chimera engineered according to the actual situation. Further, the promoter is selected from at least one of the RNA polymerase III promoters, and the RNA polymerase III promoter is a U6 promoter, an H1 promoter, or a 7SK promoter; and particularly optionally, the promoter is an H1 promoter.

The DNA sequence for expressing the shRNA is a DNA sequence for expressing any shRNA useful for treating diseases. Optionally, the DNA sequence for expressing any shRNA useful for treating diseases is one or more selected from SEQ ID No: 1 to SEQ ID No: 3 in the Sequence Listing. For example, it may be a DNA sequence for expressing an shRNA for treating HBV disease, a DNA sequence for expressing an shRNA for treating an HIV disease, optionally a DNA sequence for expressing an shRNA for treating Duchenne muscular dystrophy (DMD), a DNA sequence for expressing an shRNA for treating hypercholesterolemia, etc.

For example,
1) the DNA sequence for expressing any shRNA for treating the HBV disease, in which the target sequence of the shRNA may be composed of 19 to 23 nt fragments in the following DNA sequence, such as
   (1) catcctgctgctatgcctcat (SEQ ID No: 7)
   (2) aaggtatgttgcccgtttgtcc (SEQ ID No: 8)
   (3) cctattgattggaaagtatgtcaaa (SEQ ID No: 9)
   (4) tcgccaacttacaaggcctttct (SEQ ID No: 10)
   (5) tgtgctgccaactggatcct (SEQ ID No: 11)
   (6) ccgtgtgcacttcgcttcacct (SEQ ID No: 12)
   (7) ggaggctgtaggcataaattggtctgt (SEQ ID No: 13)
   (8) ggagtgtggattcgcactcct (SEQ ID No: 14)
2) The DNA sequence for expressing the shRNA for treating the HIV disease, in which the target sequence of shRNA may be composed of 19 to 23 nt fragments of the following RNA sequence, such as
   (1) aucaaugaggaagcugcagaaugg (SEQ ID No: 15)
   (2) gggaagugacauagcaggaacuacuag (SEQ ID No: 16)
   (3) uaaauaaaauaguaagaauguauagcccu (SEQ ID No: 17)
   (4) uaugggguaccugugugga (SEQ ID No: 18)
   (5) gccaauucccauacauuauugugc (SEQ ID No: 19)
   (6) uuaaauggcagucuagcagaa (SEQ ID No: 20)
   (7) accacacacaaggcuacuucccugau (SEQ ID No: 21)
   (8) acacccccuagcauuucaucac (SEQ ID No: 22)
   (9) ggauggugcuucaagcuaguaccaguu (SEQ ID No: 23)
3) The DNA sequence for expressing the shRNA for treating Duchenne muscular dystrophy (DMD), in which the target sequence of the shRNA may be composed of 19 to 23 nt fragments of the following RNA sequence, such as
   (1) ugaguaucaucgugugaaag (SEQ ID No: 24)
   (2) uccuuucaucucugggcuc (SEQ ID No: 25)
   (3) aacuuccucuuuaacagaaaagcauac (SEQ ID No: 26)
   (4) aacuuccucuuuaacagaaaagcauac (SEQ ID No: 27)
   (5) caaggaaguuggcauuucaa (SEQ ID No: 28)
4) The DNA sequence for expressing the shRNA for treating hypercholesterolemia, in which the target sequence of the shRNA is composed of 19 to 23 nt fragments of the following RNA sequence, such as
   (1) uuccgaauaaacuccaggc (SEQ ID No: 29)
   (2) aaccgcaguucuuuguagg (SEQ ID No: 30)
   (3) uugguauucagugugauga (SEQ ID No: 31)
   (4) ucaucacacugaauaccaa (SEQ ID No: 32)

The DNA sequence for expressing the shRNA may be a combination of DNA sequences simultaneously expressing two or more shRNA, and the combination may be a combination of two DNA sequences expressing shRNA or a combination of three DNA sequences expressing shRNA, but not limited to two or three DNA sequences. The two DNA sequences expressing shRNA adjacent to each other may be directly linked or linked by a linker; and the expressed shRNA-targeted therapeutic targets may be a single target or multiple targets.

The present disclosure provides a polynucleotide sequence carrying the shRNA expression cassette, in which both ends of the shRNA expression cassette are AAV-terminal inverted repeat sequences, respectively.

The AAV terminal inverted repeat sequence is selected from different serotypes of AAVs, optionally the AAV terminal inverted repeat sequence is selected from any serotype of AAVs in clades A-F or AAV1, AAV2, AAV3, AAV4, AAV5, AAV6, AAV7, AAV8, AAV9 or any of hybrid/chimeric types thereof, optionally the AAV terminal inverted repeat sequence is derived from the AAV2 serotype. Among them, the terminal inverted repeat sequences can be engineered, deleted or truncated at the corresponding positions, and the above methods can be used in combination.

In the polynucleotide sequence of the present disclosure, the sequence length between the two terminal inverted repeat sequences is 3.2 kb to 5.2 kb, optionally 3.8 kb to 5.1 kb, and even optionally 3.8 kb to 4.6 kb, 4.6 kb to 5.1 kb, especially optionally 4.6 kb. When trs in one terminal inverted repeat sequence is engineered, and the Rep protein restriction site mutation caused by insertion, deletion, or substitution cannot be efficiently cleaved, the sequence length between the two inverted repeat sequences is half of 3.2 kb to 5.2 kb, optionally half of 3.8 kb to 5.1 kb, or even optionally half of 3.8 kb to 4.6 kb or 4.6 kb to 5.1 kb, especially optionally half of 4.6 kb, i.e., 2.3 kb.

Optionally, a 5' end inverted repeat sequence in the polynucleotide sequence deletes a D sequence, and the sequence length between the two ITRs of the polynucleotide sequence may be half of 4.6 kb to 5.1 kb, or even optionally half of the 4.6 kb.

The present disclosure provides a recombinant vector plasmid carrying the above shRNA expression cassette or polynucleotide sequence.

The polynucleotide sequences described in the present disclosure may be commercially synthesized, and may be constructed into a recombinant vector plasmid by a molecular cloning method.

The recombinant vector plasmid is selected from an adeno-associated viral vector.

Optionlly, the adeno-associated virus vector is of various serotypes, such as any serotype of AAVs in clades A-F (see the publication of WO200533321), specifically AAV1 (GenBank: AF063497.1), AAV2 (GenBank: AF043303.1), AAV3 (GenBank: U48704.1), AAV4 (GenBank: NC_001829), AAV5 (GenBank: NC_006152), AAV6 (GenBank: AF028704), AAV7 (GenBank: AF513851), AAV8 (GenBank: AF513852), AAV9 (GenBank: AY530579) or the hybrid/chimeric type thereof.

Even optionally, the adeno-associated virus vector is of the AAV2/8 type, in which a capsid protein of the adeno-associated virus vector is from serotype VIII, and a terminal inverted repeat sequence of the adeno-associated virus vector genome is from serotype II. The nucleotide sequence of the present disclosure completely matched with the base sequence of the HBV genomic target is inserted between the two terminal inverted repeat sequences to form a gene therapy drug that targets liver cells, and the expression product efficiently and specifically inhibits HBV replication.

The adeno-associated virus vector is finally packaged as a virus. When the ITRs on both sides of the polynucleotide sequence is selected from the normal AAV serotype ITR, the sequence packaged into the virus is single-stranded, and the virus is called single-stranded AAV; and when any one of ITRs causes the mutation of the Rep protein restriction site by insertion, deletion, or substitution to be unable to be efficiently cleaved, the sequence packaged into the virus is double-stranded, and the virus is called double-stranded AAV.

The present disclosure also provides an shRNA, in which the shRNA is expressed by the shRNA expression cassette or the polynucleotide sequence or the recombinant vector plasmid described above.

The present disclosure also provides a host, in which the host includes the recombinant vector plasmidof the present disclosure. The host is one or more selected from *Escherichia coli,* HEK293 cell line, HEK293T cell line, HEK293A cell line, HEK293S cell line, HEK293FT cell line, HEK293F cell line, HEK293H cell line, HeLa cell line, SF9 cell line, SF21 cell line, SF900 cell line, and BHK cell line.

The present disclosure also provides a viral particle, including the recombinant vector plasmid described above or a vector genome of the recombinant vector plasmid in a host. The viral particle is non-selectively or selectively expressed in liver tissue or liver cancer cell.

The present disclosure also relates to an isolated and engineered cell that expresses or includes the shRNA expression cassette, polynucleotide sequence, recombinant vector plasmid, and viral particle described above. The cell is an engineered cell into which a vector genome of the recombinant vector plasmid provided in the present disclosure or in a host is introduced, including prokaryotic cells and eukaryotic cells (such as fungal cells, insect cells, plant cells, animal cells), optionally mammalian cells, optionally human cells, and optionally human liver cells and stem cells.

The present disclosure also relates to tissues and organisms, such as animals, including the above cell.

The present disclosure also relates to a pharmaceutical composition including the above cell.

The present disclosure also provides a pharmaceutical composition, including an active ingredient and a pharmaceutically acceptable excipient, in which the active ingredient is one or more selected from the shRNA expression cassette, the polynucleotide sequence carrying the shRNA expression cassette, the shRNA, the recombinant vector plasmid, the viral particle or the isolated and engineered cell of the present disclosure.

The pharmaceutical composition is an injection including a pharmaceutically acceptable excipient and the above active ingredient.

The present disclosure also provides use of the shRNA expression cassette, the polynucleotide sequence carrying the shRNA expression cassette or the recombinant vector plasmid or the shRNA, the host, the virus particle or the isolated and engineered cell described above in the preparation of a medicament for prevention and treatment of hepatitis B, acquired immunodeficiency syndrome, for treatment of Duchenne muscular dystrophy (DMD), and for treatment of hypercholesterolemia.

Conventional AAV vector preparation systems can be used for packaging the recombinant vector plasmids of the present disclosure, such as a two-plasmid packaging system, a three-plasmid packaging system, a baculovirus packaging system and an AAV packaging system using Ad or HSV as a helper virus. Among them, the three-plasmid packaging system includes a plasmid pscAAV-H1-shRNA-Stuffer, a plasmid pHelper, and a plasmid pAAV-R2CX; in which the plasmid pHelper provides E2A, E4 and VA regions of adenovirus; the plasmid pAAV-R2CX provides a sequence including a rep gene and a cap gene; the plasmid pscAAV-H1-shRNA-Stuffer contains the above polynucleotide sequence.

Among them, X refers to an AAV serotype name corresponding to a source of Cap gene constituting the pAAV-R2CX recombinant vector plasmid.

The present disclosure also provides a method for preparing the viral vector of the present disclosure by using the preparation system described above.

The present disclosure has the following advantageous effects:
1. The shRNA expression cassette of the present disclosure uses AAV virus as a vector for delivery, such that shRNA can exert a long-acting effect; as compared with naked shRNA or other chemically modified shRNA, AAV selected as a vector has a lower immunogenicity, and is safe and non-pathogenic; naked shRNA has no obvious tissue cell preference in vivo, but different serotypes of AAVs have obvious tissue targeting, which can greatly improve the efficacy of shRNA drugs.
2. The shRNA expression cassette provided by the present disclosure selects a suitable stuffer sequence, such that the sequence length of shRNA expression cassette is proximate to the length of the AAV wild type genome, so that when the AAV viral vector is selected for carrying, the packaging efficiency is ensured to be the highest; when the stuffer sequence is located at the 3' end of the shRNA sequence and the expression cassette or the polynucleotide sequence carrying the same is expressed by AAV viral vector, it not only guarantees the viral packaging yield, but also greatly improves the expression level of the target gene of shRNA, thereby improving the therapeutic effect of the drug.

The disclosure will be further described in conjunction with the following drawings and the detailed description, which are not to be construed as a limitation to the present disclosure. Any equivalent substitution in the art in accordance with the content of the present disclosure belongs to the protection scope of the present disclosure.

### BRIEF DESCRIPTION OF THE DRAWINGS

Fig. 1 is a plasmid map of pSNAV2.0-H1-shRNA1.
Fig. 2 is a plasmid map of pSNAV2.0-H1-shRNA1-intron.
Fig. 3 is a plasmid map of pSNAV2.0-H1-shRNA1-intron1.
Fig. 4 is a plasmid map of pSNAV2.0-H1-shRNA1-intron2.
Fig. 5 is a plasmid map of pSNAV2.0-H1-shRNA1-intron3.
Fig. 6 is a plasmid map of pSNAV2.0-H1-shRNA1-intron4.
Fig. 7 is a plasmid map of pSNAV2.0-H1-shRNA1-intron5.
Fig. 8 is a plasmid map of pSNAV2.0-H1-shRNA1-intron6.
Fig. 9 is a plasmid map of pSNAV2.0-H1-shRNA1-intron7.
Fig. 10 is a plasmid map of pSNAV2.0-H1-shRNA1-intron8.
Fig. 11 is a plasmid map of pSC-H1-shRNA2.
Fig. 12 is a plasmid map of pSC-Hl-shRNA2-intron'.
Fig. 13 is a plasmid map of pSC-H1-shRNA2-intron1'.
Fig. 14 is a plasmid map of pSC-H1-shRNA2-intron2'.
Fig. 15 is a plasmid map of pSC-H1-shRNA2-intron3'.
Fig. 16 is a plasmid map of pSC-H1-shRNA2-intron4'.
Fig. 17 is a plasmid map of pSC-H1-shRNA2-intron5'.
Fig. 18 is a plasmid map of pSC-H1-shRNA2-intron6'.
Fig. 19 is a plasmid map of pSNAV2.0-shRNA1-shRNA3.
Fig. 20 is a plasmid map of pSNAV2.0-shRNA1-shRNA3-intron1".
Fig. 21 is a plasmid map of pSNAV2.0-shRNA1-shRNA3-intron2".
Fig. 22 is a plasmid map of pSNAV2.0-shRNA1-shRNA3-intron3".
Fig. 23 is a Quantitative real-time PCR detection result 1 of HBV in Example 2.
Fig. 24 is a Quantitative real-time PCR detection result 2 of HBV in Example 2.
Fig. 25 is a Quantitative real-time PCR detection result 3 of HBV in Example 2.
Fig. 26 is a Quantitative real-time PCR detection result 1 of HBV in Example 4.
Fig. 27 is a Quantitative real-time PCR detection result 2 of HBV in Example 4.
Fig. 28 is a Quantitative real-time PCR detection result 3 of HBV in Example 4.
Fig. 29 is a Quantitative real-time PCR detection result 1 of HBV in Example 5.
Fig. 30 is a Quantitative real-time PCR detection result 2 of HBV in Example 5.
Fig. 31 is a Quantitative real-time PCR detection result 3 of HBV in Example 5.
Fig. 32 is a detection result 1 in Example 6.
Fig. 33 is a detection result 2 in Example 6.
Fig. 34 is a detection result 3 in Example 6.
Fig. 35 is a detection result 4 in Example 6.
Fig. 36 is a detection result 5 in Example 6.
Fig. 37 is a detection result 6 in Example 6.
Fig. 38 is a detection result 7 in Example 6.
Fig. 39 is a detection result 8 in Example 6.
Fig. 40 is a detection result of HBsAg in Example 8.
Fig. 41 is a detection result of HBV DNA in Example 8.

### DETAILED DESCRIPTION

### Example 1: Effect of single-stranded AAV packaging capacity on yield

### 1. Packaging plasmid construction:

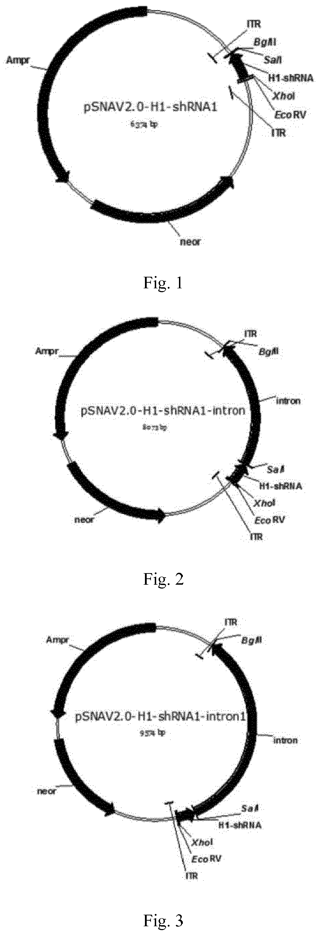

Plasmid vector construction was carried out according to "Molecular Cloning", pSNAV2.0-CMV-EGFP (purchased from Benyuan Zhengyang Gene Technology Co., Ltd., Beijing) was a shuttle plasmid used for single-stranded AAV packaging. Using HhoI and SalI as restriction sites, the H1 promoter (GenBank: X16612) and the DNA sequence for expressing shRNA1 (SEQ ID No: 1, hereinafter abbreviated as shRNA1 in the description of the plasmid) were synthesized to replace the CMV-EGFP sequence of pSNAV2.0-CMV-EGFP. The EcoRV restriction site was introduced by site-directed mutagenesis to form plasmid pSNAV2.0-H1-shRNA1, and the plasmid map is shown in Fig. 1. Plasmid construction methods are conventional methods in the art (Xiao Xiao, Juan Li, and Richard Jude Samulski. Production of high-titer recombinant adeno-associated virus vectors in the absence of helper adenovirus. J. Virol. 1998, 72(3): 2224 .).

The pSNAV2.0-H1-shRNA1 plasmid was used as the backbone plasmid, and the stuffer sequence intron was synthesized and constructed into the 3' end of the DNA sequence for expressing shRNA1 through using the restriction sites of BglII and SalI as the insertion sites to form pSNAV2.0-H1- shRNA1-intron plasmid, in which intron was derived from HPRT-intron (GenBank: M26434.1) at positions 2161-3860 (1.7 kb), and the plasmid map is shown in Fig. 2.

The pSNAV2.0-H1-shRNA1 plasmid was used as the backbone plasmid, and the stuffer sequence intron1 was synthesized and constructed into the 3' end of the DNA sequence for expressing shRNA1 through using the restriction sites of BglII and SalI as the insertion sites to form pSNAV2.0-H1- shRNA1-intron1 plasmid, in which intron1 was derived from HPRT-intron (GenBank: M26434.1) at positions 2161-5360 (3.2 kb), and the plasmid map is shown in Fig. 3.

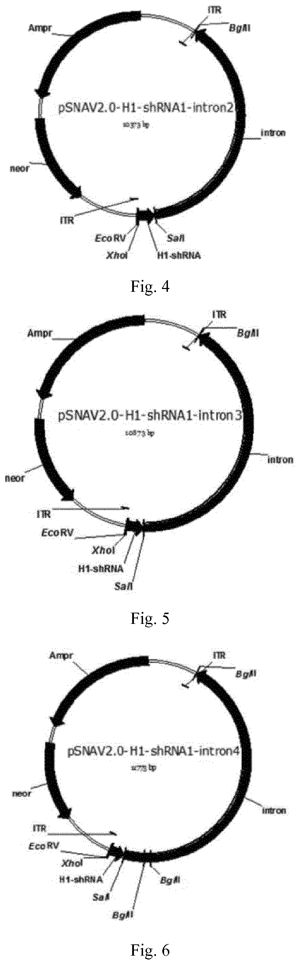

The pSNAV2.0-H1-shRNA1 plasmid was used as the backbone plasmid, and the stuffer sequence intron2 was synthesized and constructed into the 3' end of the DNA sequence for expressing shRNA1 through using the restriction sites of BglII and SalI as the insertion sites to form pSNAV2.0-H1- shRNA1-intron2 plasmid, in which intron2 was derived from HPRT-intron (GenBank: M26434.1) at positions 2161-6160 (4.0 kb), and the plasmid map is shown in Fig. 4.

The pSNAV2.0-H1-shRNA1 plasmid was used as the backbone plasmid, and the stuffer sequence intron3 was synthesized and constructed into the 3' end of the DNA sequence for expressing shRNA1 through using the restriction sites of BglII and SalI as the insertion sites to form pSNAV2.0-H1- shRNA1-intron3 plasmid, in which intron3 was derived from HPRT-intron (GenBank: M26434.1) at positions 2161-6660 (4.5 kb), and the plasmid map is shown in Fig. 5.

The pSNAV2.0-H1-shRNA1 plasmid was used as the backbone plasmid, and the stuffer sequence intron4 was synthesized and constructed into the 3' end of the DNA sequence for expressing shRNA1 through using the restriction sites of BglII and SalI as the insertion sites to form pSNAV2.0-H1- shRNA1-intron4 plasmid, in which intron4 was derived from HPRT-intron (GenBank: M26434.1) at positions 2161-7560 (5.4 kb), and the plasmid map is shown in Fig. 6.

Other packaging plasmids also include pR2C8 and AAV helper, which encode a Rep protein derived from AAV2 and a Cap protein derived from AAV8; and Ad Helper provides the minimal Ad moiety required for AAV replication. Plasmid construction methods are conventional methods in the art. (See Gao GP, Alvira MR, Wang L, Calcedo R, Johnston J, Wilson JM. Novel adeno-associated viruses from rhesus monkeys as vectors for human gene theraph. Proc Natl Acad Sci USA, 2002 Sep 3; 99(18): 11854-9).

### 2. Virus packaging and purification

The present disclosure uses HEK293 cells (purchased from ATCC) as a productioncell line, and a conventional three-plasmid packaging system to produce an AAV viral vector. The experimental methods used are all conventional methods in the art. (See Xiao Xiao, Juan Li, and Richard Jude Samulski. Production of high-titer "recombinant adeno-associated virus vectors in the absence of helper adenovirus. J. Virol. 1998, 72(3): 2224;).

### 3. Genome titer detection

An appropriate amount of purified AAV sample was taken to prepare a DNase I digestion reaction mixture according to the following table (Table 1-1). The DNase I digestion reaction mixture was incubated at 37°C for 30 min, and incubated at 75°C for 10 min, and DNase I was inactivated.

**Table 1-1**

| | |
|---|---|
| AAV sample | 5ul |
| 10×Dnase I buffer | 5ul |
| Dnase I | 1ul |
| Rnase-free water | 39ul |
| total | 50ul |

After the treated AAV purified sample was diluted by an appropriate multiple, the Q-PCR reaction system was prepared according to the following table (Table 1-2), and the detection was carried out according to the following procedure.

**Table 1-2**

| Reaction system | | Reaction procedure | |
|---|---|---|---|
| Standard/sample | 5ul | 50°C 2min | 1 cycle |
| Upstream primer (10uM) | 0.5ul | 95°C 10min | |
| Downstream primer (10uM) | 0.5ul | 95°C 15sec | 40 cycles |
| Probe (10 uM) | 0.5ul | 60°C 30sec | |
| Taqman PCR Mix (2×) | 10ul | 37°C 1sec | 1 cycle |
| ddH₂O | 3.5ul | | |

The list of promoter H1 primer probes used therein is shown as follows:

**Table 1-3**

| | | |
|---|---|---|
| Upstream primer | ATCAACCCGCTCCAAGGAAT | SEQ ID No: 4 in Sequence Listing |
| Downstream primer | AACACATAGCGACATGCAAATATTG | SEQ ID No: 5 in Sequence Listing |
| Taqman probe | CCCAGTGTCACTAGGCGGGAACACC | SEQ ID No: 6 in Sequence Listing |

The results of packaging yield are shown in Table 2-1 below:

**Table 2-1**

| Plasmid Name | Virus Vector Name | Package Length (kb) | Genome Titer (vg/ml) |
|---|---|---|---|
| pSNAV2.0-H1-shRNA1 | ssAAV2/8-H1-shRNA1 | 0.6 | 6.90E+ 10 |
| pSNAV2.0-H1-shRNA1-intron | ssAAV2/8-H1-shRNA1-intron | 2.3 | 7.20E+ 11 |
| pSNAV2.0-H1-shRNA1-in tron1 | ssAAV2/8-H1-shRNA1-in tron1 | 3.8 | 5.05E+ 12 |
| pSNAV2.0-H1-shRNA1-in tron2 | ssAAV2/8-H1-shRNA1-in tron2 | 4.6 | 7.13E+ 12 |
| pSNAV2.0-H1-shRNA1-in tron3 | ssAAV2/8-H1-shRNA1-in tron3 | 5.1 | 7.02E+ 12 |
| pSNAV2.0-H1-shRNA1-in tron4 | ssAAV2/8-H1-shRNA1-in tron4 | 6.0 | 9.41E+ 9 |

As shown in Table 2-1 above, under the circumstance having the same packaging conditions, the same package scale and the same final product volume, when the package length was in the range of 3.2 to 5.2 kb, the packaging yield could meet the demand. Specifically, when the package sequence length was proximate to the length of the wild type AAV (4.6 kb), i.e., the package length was 4.6 kb and 5.1 kb, the packaging yield was the highest; when the package sequence length was gradually lower than 3.8 kb, the packaging yield began to decrease but basically met demand; and when the package sequence length was 1.5 times the length of the wild type, the packaging yield reduced significantly.

### Example 2: Effect of the insertion position of the stuffer sequence on the expression of the target gene, taking a single-stranded AAV vector as an example

### 1. Packaging plasmid construction

The results of Example 1 demonstrate that when the package length of the single-stranded AAV virus vector was proximate to the length of the wild type AAV, the viral vector yield could be ensured. Therefore, the efficacy of different structures which were constructed by adjusting the length and position of the stuffer sequence were compared to non -stuffer insertion structure. The packaged-sequence lengths were kept 4.6 kb when stuffer sequences were inserted. For the construction of ssAAV2/8-H1-shRNA1-intron2 plasmid vector, see Example 1.

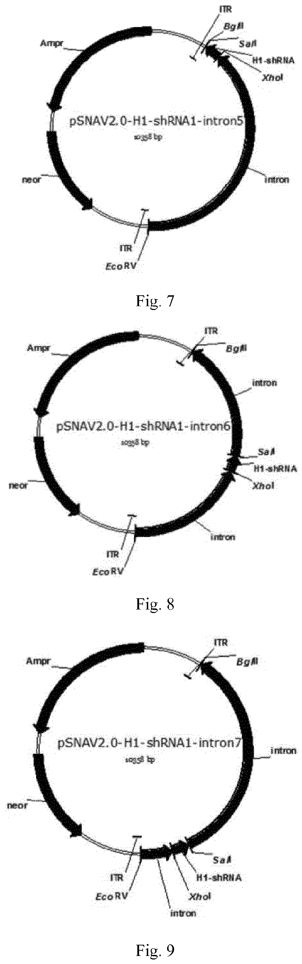

The pSNAV2.0-H1-shRNA1 plasmid was used as the backbone plasmid, and the stuffer sequence derived from HPRT-intron (GenBank: M26434.1) at positions 2161-6160 (4.0 kb) was synthesized and constructed into the 5' end of the H1 promoter sequence through using restriction sites of EcoRV and XhoI as insertion sites to form pSNAV2.0-H1-shRNA1-intron5 plasmid, and the plasmid map is shown in Fig. 7.

The pSNAV2.0-H1-shRNA1 plasmid was used as the backbone plasmid, and the stuffer sequence was synthesized segmentally. The sequence derived from HPRT-intron (GenBank: M26434.1) at positions 2161-4160 (2.0 kb) was constructed into the 5' end of the H1 promoter sequence through using the restriction sites of EcoRV and XhoI as insertion sites; and the sequence derived from HPRT-intron (GenBank: M26434.1) at positions 4161-6160 (2.0 kb) was constructed into the 3' end of the DNA sequence for expressing shRNA1 through using the restriction sites of BglII and SalI as the insertion sites to form pSNAV2.0-H1-shRNA1-intron6 plasmid, and the plasmid map is shown in Fig. 8.

The pSNAV2.0-H1-shRNA1 plasmid was used as the backbone plasmid, and the stuffer sequence was synthesized segmentally. The sequence derived from HPRT-intron (GenBank: M26434.1) at positions 2161-2660 (0.5 kb) was constructed into the 5' end of the H1 promoter sequence through using the restriction sites of EcoRV and XhoI as the insertion sites; and the sequence derived from HPRT-intron (GenBank: M26434.1) at positions 2961-5960 (3.5 kb) was constructed into the 3' end of the DNA sequence for expressing shRNA1 through using the restriction sites of BglII and SalI as the insertion sites to form pSNAV2. 0-H1-shRNA1-intron7 plasmid, and the plasmid map is shown in Fig. 9.

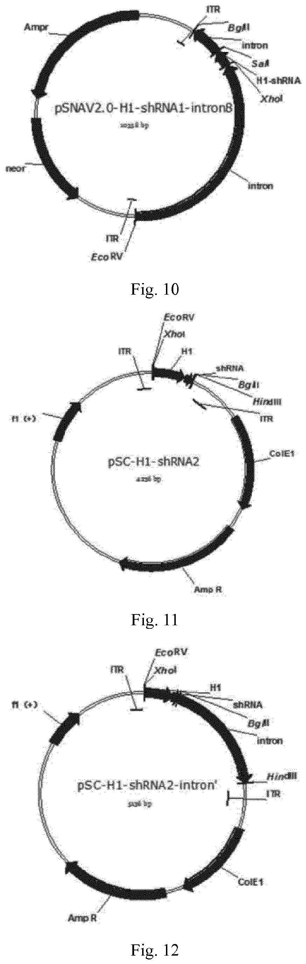

The pSNAV2.0-H1-shRNA plasmid was used as the backbone plasmid, and the stuffer sequence was synthesized segmentally. The sequence derived from HPRT-intron (GenBank: M26434.1) at positions 2161-5660 (3.5 kb) was constructed into the 5' end of the H1 promoter sequence through using the restriction sites of EcoRV and XhoI as the insertion sites; and the sequence derived from HPRT-intron (GenBank: M26434.1) at positions 5661-6160 (0.5 kb) was constructed into the 3' end of the DNA sequence for expressing shRNA1 through using the restriction sites of BglII and SalI as the insertion sites to form pSNAV2.0-H1-shRNA1-intron8 plasmid, and plasmid map is shown in Fig. 10. Other packaging plasmids further include pR2C8 and Helper, and the construction methods, please see Example 1.

### 2. Virus packaging and purification

See Example 1, "2. Virus packaging and purification".

### 3. Genome titer detection

See Example 1, "3, Genomic titer detection", and the experimental results are shown in Table 2-2.

**Table 2-2**

| Plasmid Name | Virus Vector Name | Package Length (kb) | Genome Titer (vg/ml) |
|---|---|---|---|
| pSNAV2.0-H1-shRNA1 | ssAAV2/8-H1-shRNA1 | 0.6 | 3.23E+ 11 |
| pSNAV2.0-H1-shRNA1-in tron2 | ssAAV2/8-H1-shRNA1-in tron2 | 4.6 | 7.50E+ 12 |
| pSNAV2.0-H1-shRNA1-in tron5 | ssAAV2/8-H1-shRNA1-in tron5 | 4.6 | 7.85E+ 12 |
| pSNAV2.0-H1-shRNA1-in tron6 | ssAAV2/8-H1-shRNA1-in tron6 | 4.6 | 7.64E+ 12 |
| pSNAV2.0-H1-shRNA1-in tron7 | ssAAV2/8-H1-shRNA1-in tron7 | 4.6 | 7.90E+ 12 |
| pSNAV2.0-H1-shRNA1-in tron8 | ssAAV2/8-H1-shRNA1-in tron8 | 4.6 | 7.12E+ 12 |

The results of the experiment showed that the yields of the 5 groups of AAV viral vector were comparable. As long as the length of the packaging sequence was close to the full length of the wild-type AAV genome, the insertion position of the stuffer sequence had no effect on the packaging efficiency.

### 4. Expression and detection of target shRNA

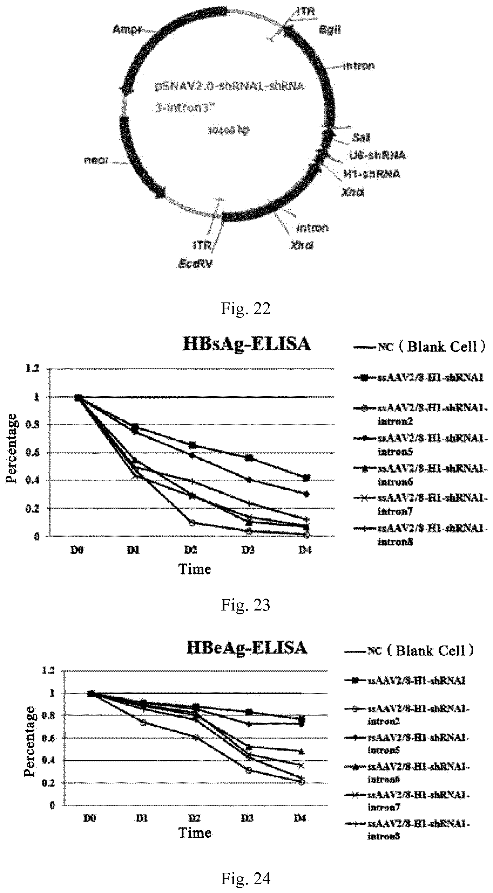

HepG2.2.15 cell infection experiments were performed with the above 5 groups of virus samples at the same multiplicity of infection (MOI), and the virus infection method was a conventional method in the art. (For specific experimental methods, see Grieger JC, Choi VW, Samulski RJ. Production and characterization of adneo-associated viral vectors. Nat Protoc. 2006; 1(3): 1412-28.). After 24 hours of infection, the supernatant of the cell culture was harvested every 24 hours. After continuous sampling for 4 times, the drug efficacy related indexes were tested. In this example, the detection indexes were HbsAg, HbeAg, and HBV DNA in the cell culture supernatant after infection, and the expression of the three indexes was compared with the three indexes of the uninfected HepG2.2.15 cell culture supernatant. The antibody detection method is a double antibody sandwich method (see the instructions of Beijing Wantai Biopharmaceutical Kit for details). The HBV DNA detection method is a Quantitative real-time PCR method, which is a conventional operation method (see the instructions of QIAGEN Test Kit for details). The results are shown in Figs. 23 to 25 and Table 2-3.

The results showed that when the stuffer sequence was all located at the 3' end of the DNA sequence for expressing shRNA1, the drug had the strongest inhibitory effect on HBV. As can be seen from the data results in the table, ssAAV2/8-H1-shRNA1-intron2 was statistically significant relative to the other examples, P < 0.01.

**Table 2-3**

| HBsAg-ELISA Detection Result | D0 | D1 | D2 | D3 | D4 |
|---|---|---|---|---|---|
| NC (blank cell) | 1 | 1 | 1 | 1 | 1 |
| ssAAV2/8-H1-shRNA1 | 1 | 0.786521 | 0.652158 | 0.56321 | 0.42153 |
| ssAAV2/8-H1-shRNA1-intron2 | 1 | 0.48058 | 0.102326 | 0.037761 | 0.017355 |
| ssAAV2/8-H1-shRNA1-intron5 | 1 | 0.752658 | 0.58173 | 0.409618 | 0.3049 |
| ssAAV2/8-H1-shRNA1-intron6 | 1 | 0.553575 | 0.299765 | 0.106104 | 0.072074 |
| ssAAV2/8-H1-shRNA1-intron7 | 1 | 0.437662 | 0.285195 | 0.142352 | 0.074649 |
| ssAAV2/8-H1-shRNA1-intron8 | 1 | 0.496668 | 0.39661 | 0.239312 | 0.124853 |

| HBeAg-ELISA Detection Result | D0 | D1 | D2 | D3 | D4 |
|---|---|---|---|---|---|
| NC (blank cell) | 1 | 1 | 1 | 1 | 1 |
| ssAAV2/8-H1-shRNA1 | 1 | 0.919418 | 0.88009 | 0.831681 | 0.769969 |
| ssAAV2/8-H1-shRNA1-intron2 | 1 | 0.744395 | 0.609283 | 0.316461 | 0.211221 |
| ssAAV2/8-H1-shRNA1-intron5 | 1 | 0.919418 | 0.86009 | 0.731681 | 0.729969 |
| ssAAV2/8-H1-shRNA1-intron6 | 1 | 0.889595 | 0.803174 | 0.524138 | 0.487348 |
| ssAAV2/8-H1-shRNA1-intron7 | 1 | 0.892424 | 0.823163 | 0.45785 | 0.361416 |
| ssAAV2/8-H1-shRNA1-intron8 | 1 | 0.857871 | 0.766679 | 0.431736 | 0.249484 |

| HBV DNA Detection Result | D0 | D1 | D2 | D3 | D4 |
|---|---|---|---|---|---|
| NC (blank cell) | 1 | 1 | 1 | 1 | 1 |
| ssAAV2/8-H1-shRNA1 | 1 | 1.024779 | 0.89806 | 0.816933 | 0.434332 |
| ssAAV2/8-H1-shRNA1-intron2 | 1 | 0.601239 | 0.533708 | 0.290499 | 0.054281 |
| ssAAV2/8-H1-shRNA1-intron5 | 1 | 0.924779 | 0.709806 | 0.616933 | 0.334332 |
| ssAAV2/8-H1-shRNA1-intron6 | 1 | 0.819469 | 0.631767 | 0.337065 | 0.158961 |
| ssAAV2/8-H1-shRNA1-intron7 | 1 | 0.794513 | 0.663432 | 0.368956 | 0.1379 |
| ssAAV2/8-H1-shRNA1-intron8 | 1 | 0.904425 | 0.556691 | 0.406397 | 0.282021 |

### Example 3: Effect of double-stranded AAV packaging capacity on yield

### 1. Packaging plasmid construction:

Plasmid vector construction was carried out according to "Molecular Cloning", pSC-CMV-EGFP was a shuttle plasmid used for double-stranded AAV packaging. Using BglII and XhoI as restriction sites, the H1 promoter and the DNA sequence for expressing shRNA2 (SEQ ID No: 2, hereinafter abbreviated as shRNA2 in the description of the plasmid) were synthesized to replace the CMV-EGFP sequence of pSC-CMV-EGFP. The EcoRV restriction site was introduced by site-directed mutagenesis to form plasmid pSC-H1-shRNA, and the plasmid map is shown in Fig. 11. The pSC-CMV-EGFP plasmid construction method is a conventional method in the art (see the corresponding pAAV-hrGFP vector construction in Wu, J, Zhao, W, Zhong, L, Han, Z, Li, B, Ma, W et al. (2007). Self-complementary recombinant adeno -associated viral vectors: packaging capacity and the role of rep proteins in vector purity. Hum Gene Ther. 2007, 18: 171-182.).

The pSC-H1-shRNA2 plasmid was used as the backbone plasmid, and the stuffer sequence intron was synthesized and constructed into the 3' end of the DNA sequence for expressing shRNA2 through using the restriction sites of BglII and HindIII as the insertion sites to form pSC-H1-shRNA2-intron' plasmid, in which intron' was derived from HPRT-intron (GenBank: M26434.1) at positions 2161- 3060 (0.9 kb), and the plasmid map is shown in Fig. 12.

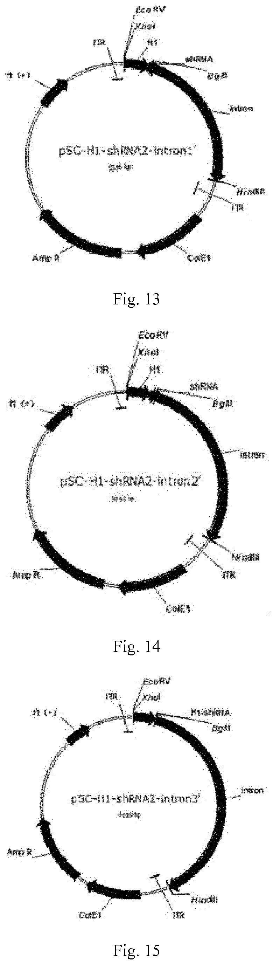

The pSC-H1-shRNA2 plasmid was used as the backbone plasmid, and the stuffer sequence intron1' was synthesized and constructed into the 3' end of the DNA sequence for expressing shRNA2 through using the restriction sites of BglII and HindIII as the insertion sites to form pSC-H1-shRNA2-intron1' plasmid, in which intron1' was derived from HPRT-intron (GenBank: M26434.1) at positions 2161-3460 (1.3 kb), and the plasmid map is shown in Fig. 13.

The pSC-H1-shRNA2 plasmid was used as the backbone plasmid, and the stuffer sequence intron2' was synthesized and constructed into the 3' end of the DNA sequence for expressing shRNA2 through using the restriction sites of BglII and HindIII as the insertion sites to form pSC-H1-shRNA2-intron2' plasmid, in which intron2' was derived from HPRT-intron (GenBank: M26434.1) at positions 2161-3860 (1.7 kb), and the plasmid map is shown in Fig. 14.

The pSC-H1-shRNA2 plasmid was used as the backbone plasmid, and the stuffer sequence intron6' was synthesized and constructed into the 3' end of the shRNA2 expression cassette through using the restriction sites of BglII and HindIII as the insertion sites to form the pSC-H1-shRNA2-intron3' plasmid, in which intron6' was derived from HPRT-intron (GenBank: M26434.1) at positions 2161- 4160 (2.0 kb), and the plasmid map is shown in Fig. 15.

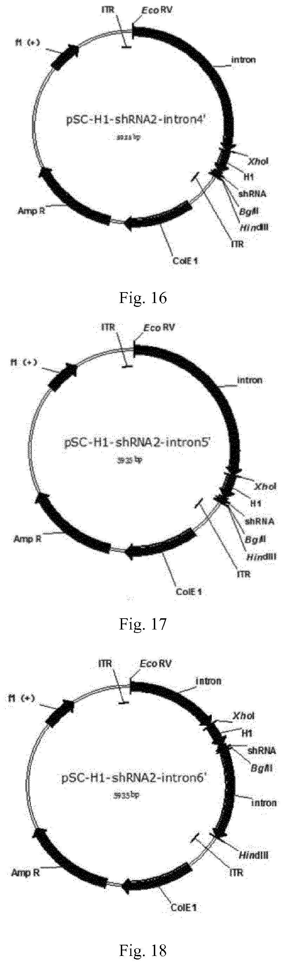

The pSC-H1-shRNA2 plasmid was used as the backbone plasmid, and the stuffer sequence intron3' was synthesized and constructed into the 3' end of the DNA sequence for expressing shRNA2 through using the restriction sites of BglII and HindIII as the insertion sites to form pSC-H1-shRNA2-intron4' plasmid, in which intron4 was derived from HPRT-intron (GenBank: M26434.1) at positions 2161- 4860 (2.7 kb), and the plasmid map is shown in Fig. 16.

Other packaging plasmids further include pR2C8 and Helper, and the construction methods, please see Example 1.

### 2. See Example 1, "2. Virus packaging and purification".

### 3. Genome titer detection

For the experimental method, see Example 1, "3. Genome titer detection". The results of packaging yield are shown in Table 2-4:

**Table 2-4**

| Plasmid Name | Virus Vector Name | Package Length (kb) | Genome Titer (vg/ml) |
|---|---|---|---|
| pSC-H1-shRNA2 | scAAV2/8-H1-shRNA2 | 0.6 | 5.20E+10 |
| pSC-H1-shRNA2-intro n' | scAAV2/8-H1-shRNA2-intr on' | 1.5 | 5.62E+11 |
| pSC-H1-shRNA2-intro n1' | scAAV2/8-H1-shRNA2-intr on1' | 1.9 | 4.56E+12 |
| pSC-H1-shRNA2-intro n2' | scAAV2/8-H1-shRNA2-intr on2' | 2.3 | 4.32E+12 |
| pSC-H1-shRNA2-intro n3' | scAAV2/8-H1-shRNA2-intr on3' | 2.6 | 4.66E+12 |
| pSC-H1-shRNA2-intro n4' | scAAV2/8-H1-shRNA2-intr on4' | 3.3 | 3.82E+10 |

The results showed that when the length of the packaging sequence was proximate to half of the length of the wild-type AAV genome, i.e., the packaging length was 1.9 kb, 2.3 kb and 2.6 kb, the packaging yield was the highest; when the length of the packaging sequence was proximate to a quarter of the length of the wild type AAV genome or less, the packaging yield began to decrease significantly; and when the length of the packaging sequence was proximate to 1.5 times the length of the wild type AAV genome, the packaging yield was also very low.

### Example 4: Effect of the insertion position of the stuffer sequence on the expression of the target gene of shRNA, taking a double-stranded AAV vector as an example

### 1. Packaging plasmid construction:

The results of Example 3 demonstrated that when the packaging length of the double-stranded AAV viral vector was proximate to half of the length of the wild-type AAV genome, and the viral vector yield could be ensured. Therefore, the efficacy of different structures which were constructed by adjusting the length and position of the stuffer sequence were compared to non -stuffer insertion structure. The packaged-sequence lengths were kept 4.6 kb when stuffer sequences were inserted. For the construction of scAAV2/8-H1-shRNA2- intron2' plasmid vector, see Example 3.

The pSC-H1-shRNA2 plasmid was used as the backbone plasmid, and a stuffer sequence derived from HPRT-intron (GenBank: M26434.1) at positions 2161-3860 (1.7 kb) was synthesized and constructed into the 5' end of the H1 promoter sequence through using the restriction sites of EcoRV and XhoI as insertion sites to form pSC-H1-shRNA2-intron5', and the plasmid map is shown in Fig. 17.

The pSC-H1-shRNA2 plasmid was used as the backbone plasmid, and the stuffer sequence was synthesized segmentally. The sequence derived from HPRT-intron (GenBank: M26434.1) at positions 2161-3010 (0.85 kb) was constructed into the 5' end of the H1 promoter sequence through using the restriction sites of EcoRV and XhoI as the insertion sites; and the sequence derived from HPRT-intron (GenBank: M26434.1) at positions 3011-3860 (0.85 kb) was constructed into the 3' end of the DNA sequence for expressing shRNA2 through using the restriction sites of BglII and SalI as the insertion sites to form pSC-H1-shRNA2-intron6' plasmid, and the plasmid map is shown in Fig. 18.

Other packaging plasmids further include pR2C8 and Helper, and the construction methods, please see Example 1.

### 2. See Example 1, "2. Virus packaging and purification".

### 3. Genome titer detection

For the detailed experimental method, see Example 1, "3. Genome titer detection". The experimental results are shown in Table 2-5.

**Table 2-5**

| Plasmid Name | Virus Vector Name | Package Length (kb) | Genome Titer (vg/ml) |
|---|---|---|---|
| pSC-H1-shRNA2 | scAAV2/8-H1-shRNA2 | 0.6 | 5.61E+12 |
| pSC-H1-shRNA2-intro n2' | scAAV2/8-H1-shRNA2-intr on2' | 2.3 | 5.50E+12 |
| pSC-H1-shRNA2-intro n5' | scAAV2/8-H1-shRNA2-intr on5' | 2.3 | 5.85E+12 |
| pSC-H1-shRNA2-intro n6' | scAAV2/8-H1-shRNA2-intr on6' | 2.3 | 5.64E+12 |

The results of the experiment showed that the yield of 3 groups of the AAV viral vectors were comparable. As long as the length of the packaging sequence was close to the full length of the wild-type AAV genome, the insertion position of the stuffer sequence had no effect on the packaging efficiency.

### 4. Expression and detection of target gene

For the experimental method, see Example 2 "4. Expression and detection of target gene".

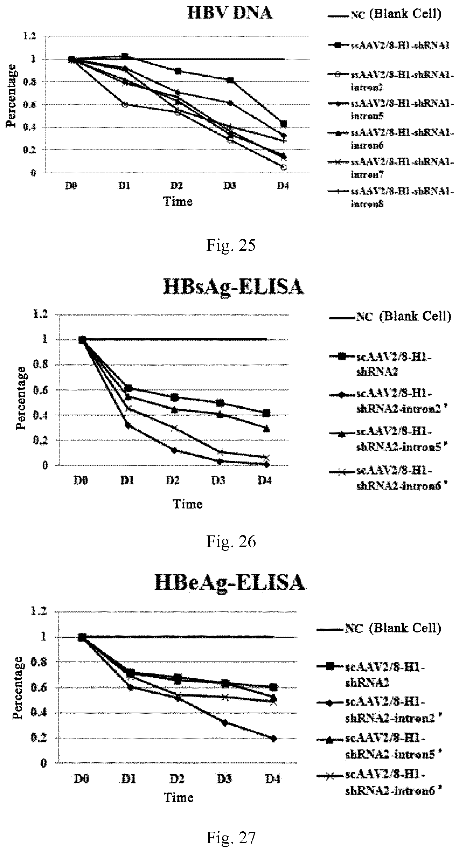

The detection results are shown in Figs. 26 to 28 and Table 2-6.

**Table 2-6**

| HbsAg-ELISA Detection Result | D0 | D1 | D2 | D3 | D4 |
|---|---|---|---|---|---|
| NC (blank cell) | 1 | 1 | 1 | 1 | 1 |
| scAAV2/8-H1-shRNA2 | 1 | 0.616521 | 0.542158 | 0.50321 | 0.42243 |
| scAAV2/8-H1-shRNA2-intron2' | 1 | 0.32058 | 0.122326 | 0.037761 | 0.0163 |
| scAAV2/8-H1-shRNA2-intron5' | 1 | 0.552658 | 0.45173 | 0.409618 | 0.304 |
| scAAV2/8-H1-shRNA2-intron6' | 1 | 0.453575 | 0.299765 | 0.106104 | 0.06134 |
| HbeAg-ELISA Detection Result | D0 | D1 | D2 | D3 | D4 |
| NC (blank cell) | 1 | 1 | 1 | 1 | 1 |
| scAAV2/8-H1-shRNA2 | 1 | 0.719418 | 0.68019 | 0.631681 | 0.6009 |
| scAAV2/8-H1-shRNA2-intron2' | 1 | 0.604395 | 0.5192 | 0.32046 | 0.198625 |
| scAAV2/8-H1-shRNA2-intron5' | 1 | 0.709418 | 0.66029 | 0.631681 | 0.529969 |
| scAAV2/8-H1-shRNA2-intron6' | 1 | 0.689595 | 0.54031 | 0.524128 | 0.48704 |

| HBV DNA Detection Result | D0 | D1 | D2 | D3 | D4 |
|---|---|---|---|---|---|
| NC (blank cell) | 1 | 1 | 1 | 1 | 1 |
| scAAV2/8-H1-shRNA2 | 1 | 0.824634 | 0.70801 | 0.516223 | 0.434332 |
| scAAV2/8-H1-shRNA2-intron2' | 1 | 0.501239 | 0.4387 | 0.240419 | 0.054281 |
| scAAV2/8-H1-shRNA2-intron5' | 1 | 0.724701 | 0.58982 | 0.51671 | 0.334332 |
| scAAV2/8-H1-shRNA2-intron6' | 1 | 0.61941 | 0.591701 | 0.327015 | 0.158961 |

The results showed that when the stuffer sequence was all located at the 3' end of the DNA sequence for expressing shRNA2, its drug had the strongest inhibitory effect on HBV. As can be seen from the data results in the table, ssAAV2/8-H1-shRNA2-intron2 was statistically significant relative to the other examples, P < 0.01.

### Example 5: Effect of the insertion position of the stuffer sequence on the expression of the target gene of a double expression cassette, taking a single-stranded AAV vector as an example

### 1. Packaging plasmid construction:

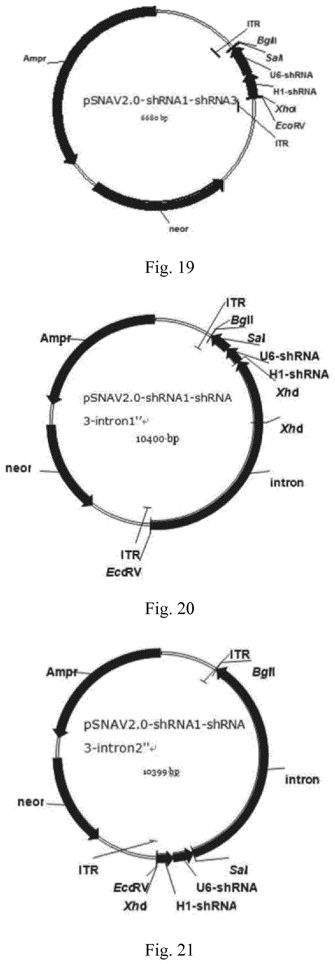

In this example, the pSNAV2.0-H1-shRNA1 was used as a backbone plasmid, and the U6 promoter and the DNA sequence for expressing shRNA3 (SEQ ID No: 3, hereinafter abbreviated as shRNA3 in the description of the plasmid) were synthesized and inserted into the 3' end of the DNA sequence for expressing shRNA3 to form the pSNAV2.0-shRNA1-shRNA3 plasmid, and the plasmid map is shown in Fig. 19. The stuffer sequence was derived from the HPRT-intron sequence at positions 2161-5880 (GenBank: M26434.1), and the length and position of the stuffer sequence were adjusted to ensure that the AAV package length was always 4.6 kb.

The pSNAV2.0-shRNA1-shRNA3 plasmid was used as the backbone plasmid, and a stuffer sequence derived from HPRT-intron (GenBank: M26434.1) at positions 2161-5880 (3.8 kb) was synthesized and constructed into the 5' end of the H1 promoter sequence through using the restriction sites of EcoRV and XhoI as insertion sites to form pSNAV2.0-shRNA1- shRNA3-intron1", and the plasmid map is shown in Fig. 20.

The pSNAV2.0-shRNA1-shRNA3 plasmid was used as the backbone plasmid, and the stuffer sequence derived from HPRT-intron (GenBank: M26434.1) at positions 2161-5880 (3.8 kb) was synthesized and constructed into the 3' end of the DNA sequence for expressing shRNA3 through using the restriction sites of BglII and SalI as the restriction sites to form pSNAV2.0-shRNA1-shRNA3-intron2" plasmid, and the plasmid map is shown in Fig. 21.

The pSNAV2.0-shRNA1-shRNA3 plasmid was used as the backbone plasmid, and the stuffer sequence was synthesized segmentally. The sequence derived from HPRT-intron (GenBank: M26434.1) at positions 2161-4020 (1.9 kb) was constructed into the 5' end of the H1 promoter sequence through using the restriction sites of EcoRV and XhoI as the insertion sites; and the sequence derived from HPRT-intron (GenBank: M26434.1) at positions 4021-5880 (1.9 kb) was constructed into the 3' end of the DNA sequence for expressing shRNA3 through using the restriction sites of BglII and SalI as the insertion sites to form pSNAV2.0-shRNA1-shRNA3-intron3", and plasmid map is shown in Fig. 22;

Other packaging plasmids further include pR2C8 and Helper, and the construction methods, please see Example 1.

### 2. See Example 1, "2. Virus packaging and purification".

### 3. Genome titer detection

For the detailed experimental method, see Example 1, "3. Genome titer detection". The experimental results are shown in Table 2-7.

**Table 2-7**

| Plasmid Name | Virus Vector Name | Package Length (kb) | Genome Titer (vg/ml) |
|---|---|---|---|
| pSNAV2.0-shRNA1-shRNA3-intron1" | ssAAV2/8-shRNA1-shRNA3-intron1" | 4.6 | 6.53E+ 12 |
| pSNAV2.0-shRNA1-shRNA3 -intron2" | ssAAV2/8-shRNA1-shRNA3 -intron2" | 4.6 | 6.82E+ 12 |
| pSNAV2.0-shRNA1-shRNA3 -intron3" | ssAAV2/8-shRNA1-shRNA3intron3" | 4.6 | 6.64E+ 12 |

The results of the experiment showed that the yield of the 3 groups of the AAV viral vectors were comparable. As long as the length of the packaging sequence was close to the full length of the wild-type AAV genome, the insertion position of the stuffer sequence had no effect on the packaging efficiency.

### 4. Expression and detection of target gene

See Example 2 "4. Expression and detection of target gene".

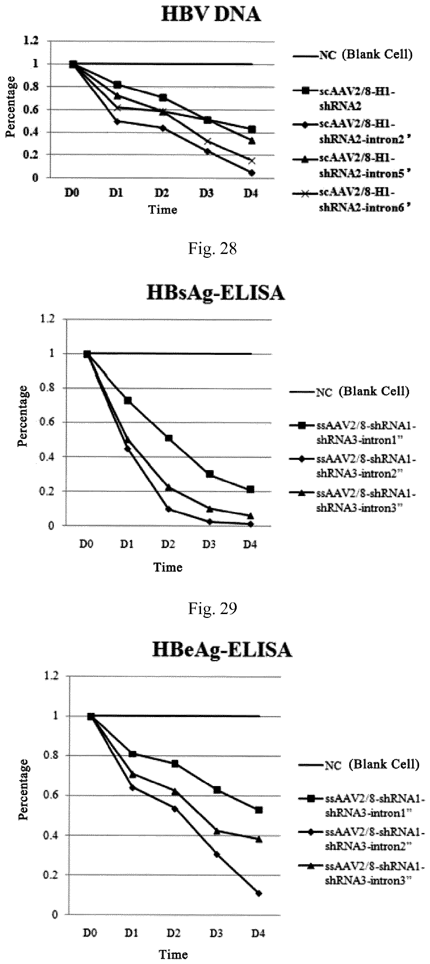

The detection results are shown in Figs. 29 to 31 and Table 2-8.

**Table 2-8**

| HbsAg-ELISA Detection Result | D0 | D1 | D2 | D3 | D4 |
|---|---|---|---|---|---|
| NC (blank cell) | 1 | 1 | 1 | 1 | 1 |
| ssAAV2/8-shRNA1-shRNA3-intron1" | 1 | 0.730654 | 0.51113 | 0.302461 | 0.211049 |
| ssAAV2/8-shRNA1-shRNA3-intron2" | 1 | 0.45057 | 0.10112 | 0.027862 | 0.015342 |
| ssAAV2/8-shRNA1-shRNA3-intron3" | 1 | 0.503505 | 0.227565 | 0.102107 | 0.062175 |

| HbeAg-ELISA Detection Result | D0 | D1 | D2 | D3 | D4 |
|---|---|---|---|---|---|
| NC (blank cell) | 1 | 1 | 1 | 1 | 1 |
| ssAAV2/8-shRNA1-shRNA3-intron1" | 1 | 0.81041 | 0.76119 | 0.630601 | 0.529762 |
| ssAAV2/8-shRNA1-shRNA3-intron2" | 1 | 0.64039 | 0.53528 | 0.30606 | 0.111025 |
| ssAAV2/8-shRNA1-shRNA3-intron3" | 1 | 0.709091 | 0.623104 | 0.42453 | 0.384341 |

| HBV DNA Detection Result | D0 | D1 | D2 | D3 | D4 |
|---|---|---|---|---|---|
| NC (blank cell) | 1 | 1 | 1 | 1 | 1 |
| ssAAV2/8-shRNA1-shRNA3-intron1" | 1 | 0.724712 | 0.6097 | 0.51153 | 0.300382 |
| ssAAV2/8-shRNA1-shRNA3-intron2" | 1 | 0.501215 | 0.433711 | 0.270494 | 0.043271 |
| ssAAV2/8-shRNA1-shRNA3-intron3" | 1 | 0.709407 | 0.581564 | 0.307165 | 0.152945 |

The results showed that when the stuffer sequence was all located at the 3' end of the DNA sequence for expressing shRNA3, the drughad the strongest inhibitory effect on HBV. As can be seen from the data results in the table, ssAAV2/8-shRNA1-shRNA3-intron2" was statistically significant relative to the other examples, P < 0.01.

### Example 6: Effect of the insertion position of the stuffer sequence on the expression of the target gene in multi-serotype AAV vector

### 1. Packaging plasmid construction:

Shuttle plasmid pSNAV2.0-H1-shRNA1-intron5 (corresponding to ssAAV-H1-shRNA1-intron5 virus packaging), pSNAV2.0-H1-shRNA1-intron2 (corresponding to ssAAV-H1-shRNA1-intron2 virus packaging), pSNAV2.0-H1-shRNA1-intron6 (corresponding to ssAAV-H1-shRNA1-intron6 virus packaging) have been constructed, see Example 2 "Packaging Plasmid Construction" for details. The structural plasmid was pR2C2, or pR2C3, or pR2C5, or pR2C7, or pR2MH31, or pR2MH39, or pR2MH43, or pR2MH47, or pR2MH31. The different serotype-derived cap genes were synthesized products, thus pR2C8 plasmid cap gene were replaced (where MH31, MH39, MH43, MH47 were from US9186419). A helper plasmid is also needed and its construction methods is the same as Example 1

### 2. See Example 1, "2. Virus packaging and purification".

### 3. Genome titer detection

For the experimental method, see Example 1, "3. Genome titer detection". The experimental results are shown in Table 2-9.

**Table 2-9**

| Virus Vector Name | Package Length (kb) | Genome Titer (vg/ml) |
|---|---|---|
| ssAAV2/2-H1-shRNA1-intron5 | 4.6 | 6.50E+12 |
| ssAAV2/2-H1-shRNA1-intron2 | 4.6 | 6.85E+12 |
| ssAAV2/2-H1-shRNA1-intron6 | 4.6 | 6.64E+12 |
| ssAAV2/3-H1-shRNA1-intron5 | 4.6 | 6.95E+12 |
| ssAAV2/3-H1-shRNA1-intron2 | 4.6 | 6.10E+12 |
| ssAAV2/3-H1-shRNA1-intron6 | 4.6 | 6.94E+12 |
| ssAAV2/5-H1-shRNA1-intron5 | 4.6 | 6.40E+12 |
| ssAAV2/5-H1-shRNA1-intron2 | 4.6 | 6.30E+12 |
| ssAAV2/5-H1-shRNA1-intron6 | 4.6 | 6.60E+12 |
| ssAAV2/7-H1-shRNA1-intron5 | 4.6 | 6.43E+12 |
| ssAAV2/7-H1-shRNA1-intron2 | 4.6 | 6.11E+12 |
| ssAAV2/7-H1-shRNA1-intron6 | 4.6 | 6.17E+12 |
| ssAAV2/MH31-H1-shRNA1-intron5 | 4.6 | 6.51E+12 |
| ssAAV2/MH31-H1-shRNA1-intron2 | 4.6 | 6.75E+12 |
| ssAAV2/MH31-H1-shRNA1-intron6 | 4.6 | 6.44E+12 |
| ssAAV2/MH39-H1-shRNA1-intron5 | 4.6 | 6.55E+12 |
| ssAAV2/MH39-H1-shRNA1-intron2 | 4.6 | 6.18E+12 |
| ssAAV2/MH39-H1-shRNA1-intron6 | 4.6 | 6.24E+12 |
| ssAAV2/MH43-H1-shRNA1-intron5 | 4.6 | 6.43E+12 |
| ssAAV2/MH43-H1-shRNA1-intron2 | 4.6 | 6.39E+12 |
| ssAAV2/MH43-H1-shRNA1-intron6 | 4.6 | 6.67E+12 |
| ssAAV2/MH47-H 1 -shRNA-intron5 | 4.6 | 6.34E+12 |
| ssAAV2/MH47-H1-shRNA1-intron2 | 4.6 | 6.81E+12 |
| ssAAV2/MH47-H1-shRNA1-intron6 | 4.6 | 6.67E+12 |

The experiment results show that the yield of AAV viral vectors in the same serotype of 3 groups were comparable. As long as the length of the packaging sequence was proximate to the full length of the wild-type AAV genome, the insertion position of the stuffer sequence had no effect on the packaging efficiency.

### 4. Expression and detection of target gene

See Example 2 "4. Expression and detection of target gene".

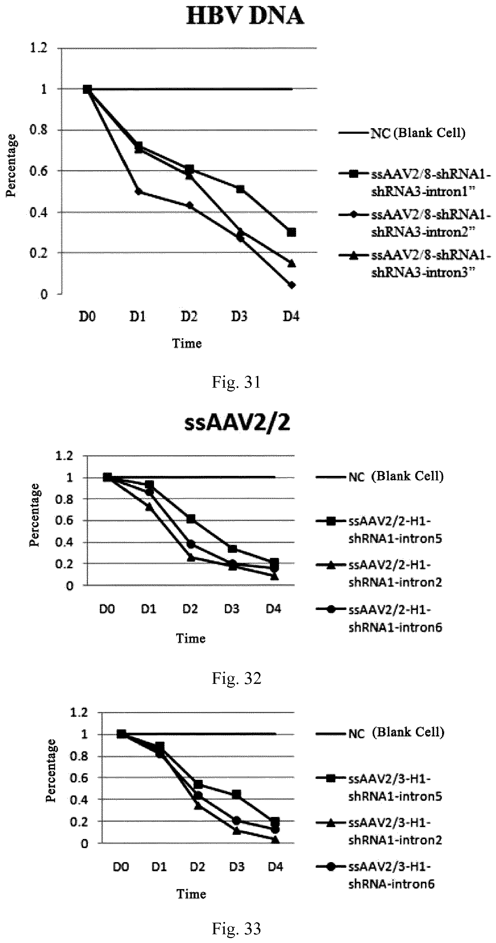

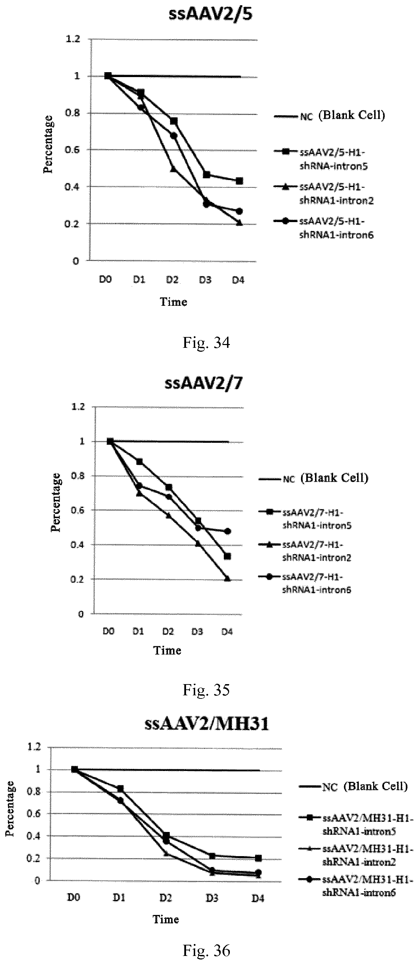

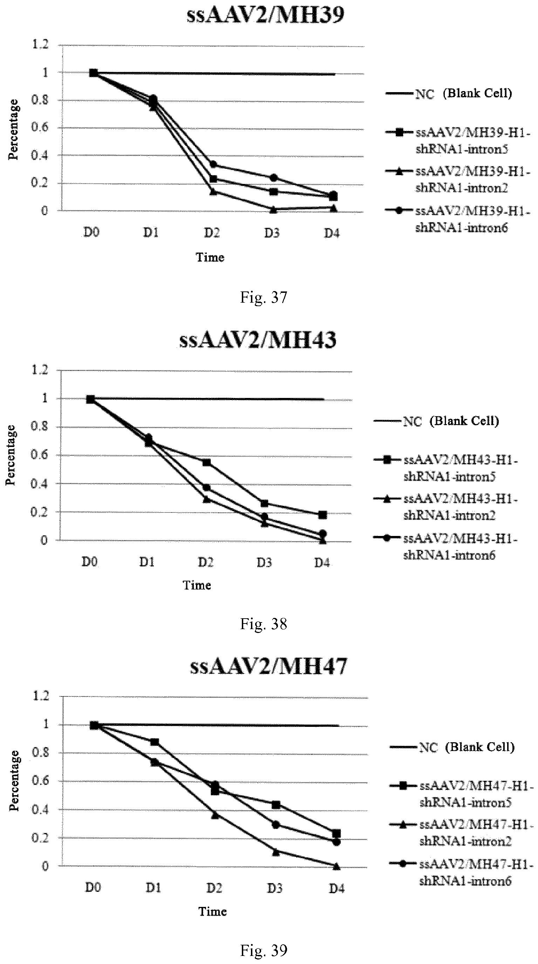

The detection results are shown in Figs. 31 to 38 and Table 2-10.

**Table 2-10**

| HbsAg-ELISA Detection Result | D0 | D1 | D2 | D3 | D4 |
|---|---|---|---|---|---|
| NC (blank cell) | 1 | 1 | 1 | 1 | 1 |
| ssAAV2/2-H1-shRNA1-intron5 | 1 | 0.930675 | 0.610601 | 0.333202 | 0.203432 |
| ssAAV2/2-H1-shRNA1-intron2 | 1 | 0.730533 | 0.259904 | 0.178197 | 0.084962 |
| ssAAV2/2-H 1 -shRNA 1 -intron6 | 1 | 0.862387 | 0.380989 | 0.20192 | 0.15791 |
| ssAAV2/3-H1-shRNA1-intron5 | 1 | 0.88453 | 0.535058 | 0.442984 | 0.188561 |
| ssAAV2/3-H1-shRNA1-intron2 | 1 | 0.852413 | 0.345115 | 0.116186 | 0.033372 |
| ssAAV2/3-H1-shRNA-intron6 | 1 | 0.817118 | 0.43886 | 0.207146 | 0.124495 |
| ssAAV2/5-H 1-shRNA-intron5 | 1 | 0.909219 | 0.75665 | 0.468068 | 0.434745 |
| ssAAV2/5-H1-shRNA1-intron2 | 1 | 0.893479 | 0.499484 | 0.332277 | 0.211844 |
| ssAAV2/5-H1-shRNA1-intron6 | 1 | 0.829574 | 0.676569 | 0.311333 | 0.272052 |
| ssAAV2/7-H1-shRNA1-intron5 | 1 | 0.88453 | 0.735058 | 0.542984 | 0.338561 |
| ssAAV2/7-H1-shRNA1-intron2 | 1 | 0.700769 | 0.57327 | 0.411992 | 0.212092 |
| ssAAV2/7-H 1 -shRNA 1 -intron6 | 1 | 0.742381 | 0.680966 | 0.50184 | 0.482693 |
| ssAAV2/MH31-H1-shRNA1-intron5 | 1 | 0.83067 | 0.412621 | 0.233202 | 0.213435 |
| ssAAV2/MH31-H1-shRNA1-intron2 | 1 | 0.730531 | 0.25152 | 0.078197 | 0.054962 |
| ssAAV2/MH31-H1-shRNA1-intron6 | 1 | 0.722307 | 0.36095 | 0.10195 | 0.082791 |
| ssAAV2/MH39-H1-shRNA1-intron5 | 1 | 0.78453 | 0.235058 | 0.142984 | 0.108561 |
| ssAAV2/MH39-H1-shRNA1-intron2 | 1 | 0.752401 | 0.145115 | 0.017156 | 0.030071 |
| ssAAV2/MH39-H1-shRNA1-intron6 | 1 | 0.81711 | 0.33886 | 0.247142 | 0.12175 |
| ssAAV2/MH43-H1-shRNA1-intron5 | 1 | 0.699219 | 0.55665 | 0.268068 | 0.189474 |
| ssAAV2/MH43-H1-shRNA1-intron2 | 1 | 0.693479 | 0.299484 | 0.132277 | 0.011844 |
| ssAAV2/MH43-H1-shRNA1-intron6 | 1 | 0.729574 | 0.376569 | 0.171333 | 0.053205 |
| ssAAV2/MH47-H1-shRNA1-intron5 | 1 | 0.88453 | 0.53515 | 0.442984 | 0.238501 |
| ssAAV2/MH47-H1-shRNA1-intron2 | 1 | 0.740769 | 0.37121 | 0.111992 | 0.012152 |
| ssAAV2/MH47-H1-shRNA1-intron6 | 1 | 0.742381 | 0.58191 | 0.30184 | 0.182623 |

The results showed that as compared with the 3 structural AAV viral vectors in the same serotype, when the stuffer sequence was all located at the 3' end of the DNA sequence for expressing shRNA1, the drug had the strongest inhibitory effect on HBV. As can be seen from the data results, the drug with a structure in which the stuffer sequence was all located at the 3' end of the shRNA was statistically significant relative to the other examples, P < 0.01.

### Example 7: Preparation method of two viral vectors

The present disclosure also provides two other methods for packaging the virus, and the AAV viral vector when packaged is not limited to the above packaging methods.
1. Adenovirus (Adv)-assisted packaging method: the packaging system must include pSNAV2.0-H1-shRNA1, pAAV2/8, and Ad5 virus solution. The cell line used in the packaging method was HeLa cells. After 24 hours of transfection with the two plasmids, the cells were auxiliary infected with Ad5 to obtain ssAAV2/8-H1-shRNA1. (See, in particular, JY Dong, PD Fan, Raymond A, Frizzell. Quantitative analysis of the packaging capacity of recombinant adeno-associated virus. Human Gene Therapy, 1996, 7:2101-2112. and Example 1). ssAAV2/8-H1-shRNA1-intron, ssAAV2/8-H1-shRNA1-intron1, ssAAV2/8-H1-shRNA1-intron2, ssAAV2/8-H1-shRNA1-intron3, ssAAV2/8-H1-shRNA1- Intron4, and ssAAV2/8-H1-shRNA1 were sequentially synthesized according to the same method. See Example 1 for subsequent viral purification and detection methods.
2. Baculovirus packaging system: this method needs to construct three plasmids pFBD-cap8, pFBD-rep2, pFB-shRNA1 (pFB-shRNA1 original vector pFB-EGFP, engineered according to "Molecular Cloning") respectively. They were transformed into DH10Bac competent cells to form three bacmid Bac-cap8, Bac-rep2, and Bac-GFP. Then, SF9 cells were infected respectively to obtain a baculovirus containing three gene expression cassettes. SF9 cells were simultaneously infected with three baculoviruses to produce ssAAV2/8-H1-shRNA1. (For specific methods, see Haifeng Chen. Intron splicing-mediated expression of AAV rep and cap genes and production of AAV vectors in insect cells. Mol Ther. 2008 May; 16(5): 924-30. and Example 1). ssAAV2/8-H1-shRNA1-intron, ssAAV2/8-H1-shRNA1-intron1, ssAAV2/8-H1-shRNA1-intron2, ssAAV2/8-H1-shRNA1-intron3, ssAAV2/8-H1-shRNA1- Intron4, and ssAAV2/8-H1-shRNA1 were sequentially synthesized according to the same method. See Example 1 for subsequent viral purification and detection methods.

This method can also be modified to construct cap and rep into the same baculovirus, and finally form two baculovirus co-infections to achieve the purpose of producing AAV.

**Table 2-11**

| Virus Vector Name | Packing Method | Package Length (kb) | Genome Titer (vg/ml) |
|---|---|---|---|
| ssAAV2/8-H1-shRNA1 | Ad-assisted two plasmids packaging | 0.6 | 6.6E+9 |
| ssAAV2/8-H1-shRNA1-intron | Ad-assisted two plasmids packaging | 2.3 | 6.03E+10 |
| ssAAV2/8-H1-shRNA1-intron1 | Ad-assisted two plasmids packaging | 3.8 | 6.25E+11 |
| ssAAV2/8-H1-shRNA1-intron2 | Ad-assisted two plasmids packaging | 4.6 | 6.78E+11 |
| ssAAV2/8-H1-shRNA1-intron3 | Ad-assisted two plasmids packaging | 5.1 | 6.62E+11 |
| ssAAV2/8-H1-shRNA1-intron4 | Ad-assisted two plasmids packaging | 6.0 | 5.99E+8 |
| ssAAV2/8-H1-shRNA1 | Baculovirus packaging | 0.6 | 7.12E+11 |
| ssAAV2/8-H1-shRNA1-intron | Baculovirus packaging | 2.3 | 7.65E+12 |
| ssAAV2/8-H1-shRNA1-intron1 | Baculovirus packaging | 3.8 | 7.44E+13 |
| ssAAV2/8-H1-shRNA1-intron2 | Baculovirus packaging | 4.6 | 7.32E+13 |
| ssAAV2/8-H1-shRNA1-intron3 | Baculovirus packaging | 5.1 | 7.58E+13 |
| ssAAV2/8-H1-shRNA1-intron4 | Baculovirus packaging | 6.0 | 6.95E+10 |

The above experimental results showed that no matter which virus packaging system was selected, the appropriate length of the stuffer sequence was selected, such that the length of the packaging sequence was proximate to the length of the AAV wild type genome, that was 3.8 kb to 5.1 kb, and the high packaging efficiency could be ensured.

### Example 8: In vivo pharmacodynamic experiment

Two drugs having two structures scAAV2/8-H1-shRNA2-intron2 and scAAV2/8-H1-shRNA2-intron5 were compared with the commercial drugs lamivudine and entecavir for evaluating in vivo efficacy. The administration mode was intravenous injection, and 14 days was a blood collection period. After separating serum samples, HBsAg and HBV DNA were detected. The grouping of animal experiments is shown as follows:

| Group | Drug administration | Dose (vg/animal) | HBV transgenic BALB/c Number of animals (n) |
|---|---|---|---|
| 1 | DPBS | 0.2 ml/animal | 6 |
| 2 | Lamivudine (LAM) | 150 mg/kg | 6 |
| 3 | Entecavir (ETV) | 3.2mg/kg | 6 |
| 4 | scAAV2/8-H1-shRNA2-intron2 | 3×10¹⁰ | 6 |
| 5 | scAAV2/8-H1-shRNA2-intron5 | 1.5×10¹¹ | 6 |

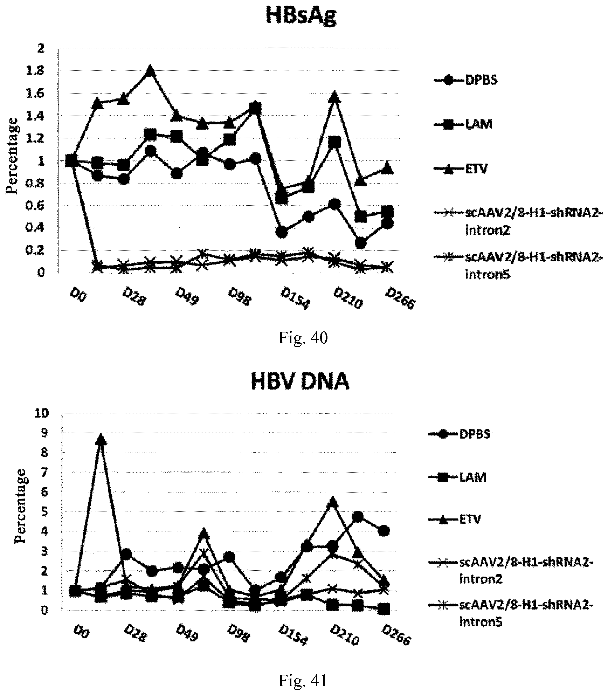

The experiment was conducted for a total of 266 days (38 weeks). For the experimental method, see Example 2 "4. Expression and detection of target gene". The detection results are shown in Fig. 40 and Table 2-12 (HBsAg detection), and Fig. 41 and Table 2-13 (HBV DNA detection).

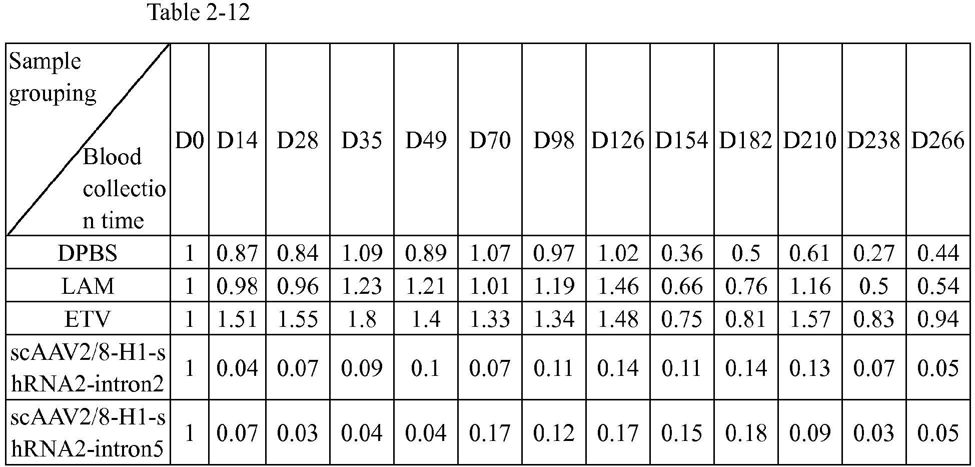

**Table 2-13**

| | D0 | D14 | D28 | D35 | D49 | D70 | D98 | D126 | D154 | D182 | D210 | D238 | D266 |
|---|---|---|---|---|---|---|---|---|---|---|---|---|---|
| DPBS | 1 | 1.13 | 2.85 | 2 | 2.18 | 2.1 | 2.72 | 1.02 | 1.68 | 3.23 | 3.27 | 4.77 | 4.02 |
| LAM | 1 | 0.69 | 0.87 | 0.72 | 0.68 | 1.26 | 0.41 | 0.24 | 0.56 | 0.8 | 0.28 | 0.26 | 0.07 |
| ETV | 1 | 8.68 | 1.24 | 1.08 | 1.25 | 3.93 | 1.05 | 0.72 | 1.06 | 3.33 | 5.5 | 2.96 | 1.55 |
| scAAV2/8-H1-sh RNA2-intron2 | 1 | 1.17 | 1.58 | 0.8 | 0.57 | 1.71 | 0.51 | 0.32 | 0.42 | 0.8 | 1.11 | 0.88 | 1.04 |
| scAAV2/8-H1-sh RNA2-intron5 | 1 | 0.7 | 1.02 | 0.96 | 1.21 | 2.9 | 0.63 | 0.56 | 0.55 | 1.63 | 2.87 | 2.36 | 1.2 |

As can be seen from the above, when the dose of scAAV2/8-H1-shRNA2-intron5 was 5 times that of scAAV2/8-H1-shRNA2-intron2, the two drugs had equivalent levels of inhibition on HBsAg in the serum of model animals. After D14, the inhibitory effect reached the strongest, and the inhibition lasted until D266 days. The pharmacodynamic effects of the two drugs having two structures were stronger than those of the commercial drugs, in which the drug having the structure of scAAV2/8-H1-shRNA2-intron2 had the same level of inhibition on HBV DNA in the serum of the model animal as the dose of lamivudine, and the drug having the structure of scAAV2/8-H1-shRNA2-intron5 had a slightly lower level of inhibition on HBV DNA in the serum of the model animal.

## Claims

1. An shRNA expression cassette, wherein in an order of 5' to 3', the shRNA expression cassette sequentially comprises a DNA sequence for expressing the shRNA and a stuffer sequence, and a sequence length of the shRNA expression cassette is proximate to a length of a wild-type AAV genome; and the stuffer sequence is optionally a human non-coding sequence.

2. The shRNA expression cassette of claim 1, wherein the human non-coding sequence is selected from a sequence fragment or a combination of a plurality of sequence fragments of an intron sequence of human factor IX, a sequence of human cosmid C346 or an HPRT-intron sequence.

3. The shRNA expression cassette of claim 1 or 2, wherein the human non-coding sequence is a sequence fragment of the HPRT-intron sequence.

4. The shRNA expression cassette of any one of claims 1 to 3, wherein the sequence length of the shRNA expression cassette that is expressed by using a single-stranded AAV viral vector or used directly is 3.2 kb to 5.2 kb, optionally 3.8 kb to 5.1 kb.

5. The shRNA expression cassette of any one of claims 1 to 3, wherein the sequence length of the shRNA expression cassette that is expressed by using a double-stranded AAV viral vector is half of the sequence length of the shRNA expression cassette that is expressed by using the single-stranded AAV viral vector or used directly.

6. The shRNA expression cassette of any one of claims 1 to 5, wherein a 5' end of the DNA sequence for expressing the shRNA in the shRNA expression cassette comprises a promoter.

7. The shRNA expression cassette of claim 6, wherein the promoter comprises one or more selected from an RNA polymerase II promoter and an RNA polymerase III promoter.

8. The shRNA expression cassette of claim 7, wherein the RNA polymerase II promoter is a tissue-specific promoter; optionally, the RNA polymerase II promoter is a liver-specific promoter; optionally the liver-specific promoter is LP1 promoter, ApoE/hAAT promoter, DC172 promoter, DC190 promoter, ApoA-I promoter, TBG promoter, LSP1 or HD-IFN promoter.

9. The shRNA expression cassette of claim 7, wherein the RNA polymerase III promoter is a U6 promoter, an H1 promoter, or a 7SK promoter; optionally, the RNA polymerase III promoter is an H1 promoter.

10. The shRNA expression cassette of any one of claims 1 to 9, wherein the DNA sequence for expressing the shRNA is a DNA sequence for expressing any shRNA useful for treating diseases; optionally, the DNA sequence for expressing any shRNA useful for treating diseases is one or more selected from SEQ ID No: 1 to SEQ ID No: 3 in the Sequence Listing.

11. A polynucleotide sequence, carrying the shRNA expression cassette of any one of claims 1 to 10, wherein both ends of the shRNA expression cassette are Adeno-Associated Virus (AAV) terminal inverted repeat sequences, respectively.

12. The polynucleotide sequence of claim 11, wherein the AAV terminal inverted repeat sequence is selected from different serotypes of AAVs, optionally the AAV terminal inverted repeat sequence is selected from any serotype of AAVs in clades A-F or AAV1, AAV2, AAV3, AAV4, AAV5, AAV6, AAV7, AAV8, AAV9 or any of hybrid/chimeric types thereof, optionally the AAV terminal inverted repeat sequence is derived from the AAV2 serotype.

13. The polynucleotide sequence of claim 12, wherein the sequence length between the two terminal inverted repeat sequences in the polynucleotide sequence is optionally 3.2 kb to 5.2 kb; when trs in one terminal inverted repeat sequence is engineered, and a Rep protein cleavage site mutation caused by insertion, deletion, or substitution cannot be efficiently cleaved, the sequence length between the two inverted repeat sequences is optionally half of 3.2 kb to 5.2 kb.

14. The polynucleotide sequence of any one of claims 11 to 13, wherein a 5' end inverted repeat sequence in the polynucleotide sequence deletes a D sequence, and the sequence length between the two terminal inverted repeat sequences in the polynucleotide sequence is optionally half of 4.6 kb to 5.1 kb.

15. A recombinant vector plasmid, carrying the shRNA expression cassette of any one of claims 1 to 10 or the polynucleotide sequence of any one of claims 11 to 14.

16. The recombinant vector plasmid of claim 15, wherein the recombinant vector plasmid is an adeno-associated virus vector.

17. The recombinant vector plasmid of claim 16, wherein the adeno-associated virus vector is of the AAV2/8 type, in which a capsid protein of the adeno-associated virus vector is from serotype VIII, and a terminal inverted repeat sequence of the adeno-associated virus vector is from serotype II.

18. An shRNA, expressed by the shRNA expression cassette of any one of claims 1 to 10, or the polynucleotide sequence of any one of claims 11 to 14, or the recombinant vector plasmid of any one of claims 15 to 17.

19. A host comprising the recombinant vector plasmid of any one of claims 15 to 17.

20. The host of claim 19, wherein the host is selected from one or more of *Escherichia coli,* HEK293 cell line, HEK293T cell line, HEK293A cell line, HEK293S cell line, HEK293FT cell line, HEK293F cell line, HEK293H cell line, HeLa cell line, SF9 cell line, SF21 cell line, SF900 cell line, and BHK cell line.

21. A viral particle, comprising the recombinant vector plasmid of any one of claims 15 to 17, or a vector genome of the recombinant vector plasmid of the host of any one of claims 19 to 20.

22. The viral particle of claim 21, wherein the viral particle is non-selectively or selectively expressed in liver tissue or liver cancer cell.

23. An isolated and engineered cell, wherein the cell expresses or comprises the shRNA expression cassette of any one of claims 1 to 10, the polynucleotide sequence of any one of claims 11 to 14, the recombinant vector plasmid of any one of claims 15 to 17, or the viral particle of claim 21 or 22.

24. The engineered cell of claim 23, wherein the cell is a mammalian cell, optionally a human cell, or even optionally a human stem cell or hepatocyte.

25. A pharmaceutical composition, comprising an active ingredient and a pharmaceutically acceptable excipient, wherein the active ingredient is selected from one or more of the shRNA expression cassette of any one of claims 1 to 10, the polynucleotide sequence of any one of claims 11 to 14, the recombinant vector plasmid of any one of claims 15 to 17, the shRNA of claim 18, the viral particle of claim 21 or 22, and the isolated and engineered cell of claim 23 or 24.

26. The pharmaceutical composition of claim 25, wherein the pharmaceutical composition is an injection comprising a pharmaceutically acceptable excipient and the active ingredient.

27. Use of the shRNA expression cassette of any one of claims 1 to 10, the polynucleotide sequence of any one of claims 11 to 14, the recombinant vector plasmid of any one of claims 15-17, the shRNA of claim 18, the virus particle of claim 21 or 22, or the isolated and engineered cell of claim 23 or 24 in preparation of a medicament for prevention and treatment of hepatitis B, acquired immunodeficiency syndrome, for treatment of Duchenne muscular dystrophy (DMD), and for treatment of hypercholesterolemia.

28. A vector preparation system for packaging the recombinant vector plasmid of claims 15 to 17, wherein the vector preparation system is a conventional AAV vector preparation system comprising a two-plasmid packaging system, a three-plasmid packaging system, a baculovirus packaging system, and an AAV packaging system using Ad or HSV as a helper virus.

29. The vector preparation system of claim 28, wherein the three-plasmid packaging system comprises a plasmid pscAAV-H1-shRNA-Stuffer, a plasmid pHelper, and a plasmid pAAV-R2CX; wherein
the plasmid pHelper provides E2A, E4 and VA regions of adenovirus;
the plasmid pAAV-R2CX provides a sequence comprising a rep gene and a cap gene;
the plasmid pscAAV-H1-shRNA-Stuffer comprises the polynucleotide sequence of any one of claims 11 to 14,
X refers to an AAV serotype name corresponding to a source of Cap gene constituting the pAAV-R2CX recombinant vector plasmid.


Sequence Listing

<110>  STAIDSON (BEIJING) BIOPHMACEUTICAL CO., LTD.
       BEIJING SOLOBIOGENETECHNOLOGY COMPANY LTD.

<120>  SHRNA EXPRESSION CASSETTE, POLYNUCLEOTIDE SEQUENCE CARRYING THE SAME,
AND USE THEREOF

<130>  PCT180159CN

<160>  32

<170>  PatentIn version 3.5

<210>  1
<211>  51
<212>  DNA
<213>  Artificial Sequence

<400>  1
cctattgatt ggaaagtatg tttcaagaga acatactttc caatcaatag g          51


<210>  2
<211>  47
<212>  DNA
<213>  Artificial Sequence

<400>  2
gtgtgcactt cgcttcacct tcaagagagg tgaagcgaag tgcacac               47


<210>  3
<211>  47
<212>  DNA
<213>  Artificial Sequence

<400>  3
ggtatgttgc ccgtttgtct tcaagagaga caaacgggca acatacc               47


<210>  4
<211>  20
<212>  DNA
<213>  Artificial Sequence, Primer

<400>  4
atcaacccgc tccaaggaat                                             20


<210>  5
<211>  25
<212>  DNA
<213>  Artificial Sequence, Primer

<400>  5
aacacatagc gacatgcaaa tattg                                       25


<210>  6
<211>  25
<212>  DNA
<213>  Artificial Sequence, Probe

<400>  6
cccagtgtca ctaggcggga acacc                                            25


<210>  7
<211>  21
<212>  DNA
<213>  Artificial Sequence

<400>  7
catcctgctg ctatgcctca t                                                21


<210>  8
<211>  22
<212>  DNA
<213>  Artificial Sequence

<400>  8
aaggtatgtt gcccgtttgt cc                                               22


<210>  9
<211>  25
<212>  DNA
<213>  Artificial Sequence

<400>  9
cctattgatt ggaaagtatg tcaaa                                            25


<210>  10
<211>  23
<212>  DNA
<213>  Artificial Sequence

<400>  10
tcgccaactt acaaggcctt tct                                              23


<210>  11
<211>  20
<212>  DNA
<213>  Artificial Sequence

<400>  11
tgtgctgcca actggatcct                                                  20


<210>  12
<211>  22
<212>  DNA
<213>  Artificial Sequence

<400>  12
ccgtgtgcac ttcgcttcac ct                                               22


<210>  13
<211>  27
<212>  DNA
<213>  Artificial Sequence

<400>  13
ggaggctgta ggcataaatt ggtctgt                                          27


<210>  14
<211>  21
<212>  DNA
<213>  Artificial Sequence

<400>  14
ggagtgtgga ttcgcactcc t                                                21


<210>  15
<211>  24
<212>  RNA
<213>  Artificial Sequence

<400>  15
aucaaugagg aagcugcaga augg                                             24


<210>  16
<211>  27
<212>  RNA
<213>  Artificial Sequence

<400>  16
gggaagugac auagcaggaa cuacuag                                          27


<210>  17
<211>  29
<212>  RNA
<213>  Artificial Sequence

<400>  17
uaaauaaaau aguaagaaug uauagcccu                                        29


<210>  18
<211>  19
<212>  RNA
<213>  Artificial Sequence

<400>  18
uaugggguac cugugugga                                                   19


<210>  19
<211>  24
<212>  RNA
<213>  Artificial Sequence

<400>  19
gccaauuccc auacauuauu gugc                                             24


<210>  20
<211>  21
<212>  RNA
<213>  Artificial Sequence

<400>  20
uuaaauggca gucuagcaga a                                                21

<210>  21
<211>  26
<212>  RNA
<213>  Artificial Sequence

<400>  21
accacacaca aggcuacuuc ccugau                                           26

<210>  22
<211>  23
<212>  RNA
<213>  Artificial Sequence

<400>  22
acagccgccu agcauuucau cac                                              23

<210>  23
<211>  27
<212>  RNA
<213>  Artificial Sequence

<400>  23
ggauggugcu ucaagcuagu accaguu                                          27

<210>  24
<211>  20
<212>  RNA
<213>  Artificial Sequence

<400>  24
ugaguaucau cgugugaaag                                                  20

<210>  25
<211>  19
<212>  RNA
<213>  Artificial Sequence

<400>  25
uccuuucauc ucugggcuc                                                   19

<210>  26
<211>  27
<212>  RNA
<213>  Artificial Sequence

<400>  26
aacuuccucu uuaacagaaa agcauac                                          27

<210>  27
<211>  27
<212>  RNA
<213>  Artificial Sequence

<400>  27
aacuuccucu uuaacagaaa agcauac                                          27


<210>  28
<211>  20
<212>  RNA
<213>  Artificial Sequence

<400>  28
caaggaaguu ggcauuucaa                                                  20


<210>  29
<211>  19
<212>  RNA
<213>  Artificial Sequence

<400>  29
uuccgaauaa acuccaggc                                                   19


<210>  30
<211>  19
<212>  RNA
<213>  Artificial Sequence

<400>  30
aaccgcaguu cuuuguagg                                                   19


<210>  31
<211>  19
<212>  RNA
<213>  Artificial Sequence

<400>  31
uugguauuca gugugauga                                                   19


<210>  32
<211>  19
<212>  RNA
<213>  Artificial Sequence

<400>  32
ucaucacacu gaauaccaa                                                   19